# EUROPÄISCHE PATENTSCHRIFT

(11) **EP 0 834 536 B1**
(45) Veröffentlichungstag und Bekanntmachung des Hinweises auf die Patenterteilung: **05.12.2001**
(21) Anmeldenummer: 97116944.6
(22) Anmeldetag: 30.09.1997
(51) Int. Cl.: C09B 61/00, C07C 403/24

(54) **Umwandlung von Xanthophyllen**
Isomerization of xanthophylls
Isomérisation de xanthophylles

(30) Priorität: 04.10.1996 EP 96115908
(43) Veröffentlichungstag der Anmeldung: 08.04.1998
(73) Patentinhaber: F. HOFFMANN-LA ROCHE AG, 4070 Basel (CH)
(72) Erfinder: Bernhard, Kurt, 4419 Lupsingen (CH); Giger, Alfred, 4313 Möhlin (CH)
(74) Vertreter: Kellenberger, Marcus, Dr.

(56) Entgegenhaltungen:
- WO-A-96/02594
- WO-A-97/31894
- FR-A- 1 455 838
- ANDREWES A G: "ISOMERIZATION OF EPSILON-CAROTENE TO BETA-CAROTENE AND OF LUTEIN TO ZEAXANTHIN" ACTA CHEMICA SCANDINAVICA SERIES B - ORGANIC CHEMISTRY AND BIOCHEMISTRY, Bd. 28, Nr. 1, 1974, Seite 137/138 XP002033437
- KARRER P ET AL: "UMWANDLUNG VON ALPHA-CAROTIN IN BETA-CAROTIN UND VON XANTHOPHYLL IN ZEAXANTHIN" HELVETICA CHIMICA ACTA, Bd. 30, Nr. 1, 1947, Seite 266/267 XP002033438
- DATABASE WPI Section Ch, Week 9617 Derwent Publications Ltd., London, GB; Class D13, AN 96-167352 XP002051422 & JP 08 048 895 A (ESPIONOSA SANCHEZ R R) , 20.Februar 1996
- R.BUCHECKER et al. Chimia 26/3 134 (1972)

## Beschreibung

Die vorliegende Erfindung betrifft ein Verfahren zur Umwandlung von Xanthophyllen, insbesondere von Lutein in Zeaxanthin.

Die Xanthophylle Lutein und Zeaxanthin gehören bekanntlich der grossen Substanzklasse Carotinoide an und sind als natürliche Pigmente in der Natur weit verbreitet. Sie kommen vor allem in höheren Pflanzen, Algen, Fischen, Crustaceen und Bakterien vor. Lutein und Zeaxanthin befinden sich oft zusammen und in veresterter Form in derselben Quelle, wobei allerdings je nach Quelle das Verhältnis Lutein zu Zeaxanthin stark variiert. So macht normalerweise Lutein den wesentlich grösseren Anteil als Zeaxanthin aus bei deren gemeinsamen Vorkommen in Pflanzen, z.B. Melonen und Sammetblumen [Tagetes (T.) erecta, T. patula und weitere Tagetes-Spezies] sowie in Algen. Als Ausnahme sei der grössere Anteil von Zeaxanthin gegenüber Lutein in Mais genannt, wobei in Maiskörnern das erstere sogar den Hauptvertreter der vorhandenen Carotinoide darstellt. Zudem hängt die Stereoisomerie von Lutein und von Zeaxanthin von der Quelle ab. In pflanzlichen Quellen kommt vor allem (3R,3'R,6'R)-Lutein bzw. (3R,3'R)-Zeaxanthin vor, während in tierischen Quellen, z.B. in Fischen und Crustaceen, Lutein sich in der (3R,3'R,6'R)-, (3R,3'R,6'S)- und (3R,3'S,6'S)-Form, und Zeaxanthin sich in der (3R,3'S)- und (3S,3'S)-Form, befindet. ,

Als Carotinoide finden Lutein und Zeaxanthin entsprechende Verwendung, insbesondere als Pigmente, z.B. für den Eidotter, die Integumente (etwa Haut, Ständer und Schnäbel) und das subkutane Fett von Geflügel, das Fleisch und die Integumente (Haut, Schuppen und Schale) von Fischen und Crustaceen sowie Lebensmittel. Es wird bei vielen Anwendungen Zeaxanthin vorzugsweise eingesetzt, da dieses bei vergleichbarer Dosierung eine intensivere goldgelbe Pigmentierung bewirkt als Lutein.

Wenn von einem die beiden Xanthophylle enthaltenden pflanzlichen Rohstoff, wie beispielsweise Tagetes erecta-Blüten oder einem gegebenenfalls im voraus verseiften Extrakt davon, als Quelle des erwünschten Zeaxanthins Gebrauch gemacht wird, ist es aus naheliegenden wirtschaftlichen Gründen zweckmässiger, womöglich zuerst das Lutein in das Zeaxanthin chemisch umzuwandeln und dann das letztere aus dem Gemisch durch Extraktion zu isolieren, als die Extraktion ohne vorige Umwandlung vorzunehmen. Es ist ebenfalls von wirtschaftlicher Bedeutung, reines oder nahezu reines Lutein in Zeaxanthin chemisch umzuwandeln.

Bekanntlich kann die Umwandlung von Lutein in Zeaxanthin unter stark basischen Bedingungen erfolgen, und es sind aus der Fachliteratur verschiedene diesbezügliche Umwandlungsverfahren bekannt geworden. Bereits im Jahre 1946 haben Karrer und Jucker die Isomerisierung von natürlichem Lutein (als "Xanthophyll" bezeichnet) zu Zeaxanthin in einem Ethanol-Benzol-Gemisch mit Natriumethanolat durch 30-stündiges Erhitzen bei 100-110°C in einem evakuierten Bombenrohr realisiert [siehe Helv. Chim. Acta 30, 266-267 (1947)]. Die Ausbeute an Zeaxanthin war jedoch äusserst bescheiden. Auf diese Weise wurde wohl zum ersten Mal Lutein, der Formel 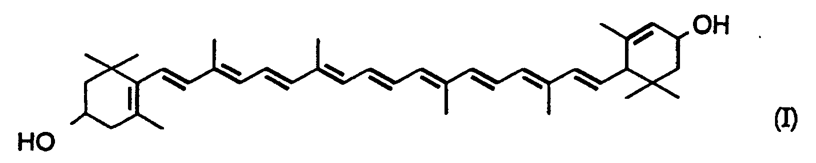 (ohne Angabe der Konfiguration) in Zeaxanthin, der Formel 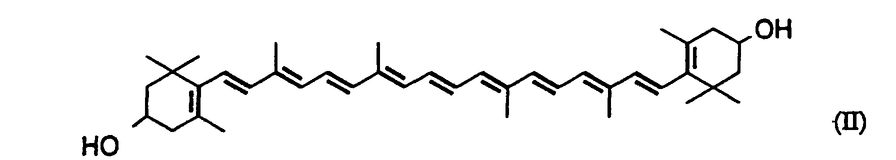 gezielt chemisch umgewandelt, wobei die isolierte Doppelbindung im ε-Ring [(I), rechts] in die Konjugation mit den restlichen Doppelbindungen des Moleküls [(II), rechts: β-Ring] gedrängt wird. Später [1959, siehe Arch. Biochem. Biophys. 88, 59-63 (1960)] ist Kargl und Quackenbush die analoge basenkatalysierte Umwandlung von δ-Carotin (ε,ψ-Carotin) in γ-Carotin (β,ψ-Carotin) gelungen. Buchecker et al. konnten jedoch die frühere Arbeit von Karrer und Jucker nicht zufriedenstellend wiederholen [Chimia 26, Nr. 3, 134-136 (1972)]. Andrewes berichtete in Acta Chem. Scand. B 28, Nr. 1, 137-138 (1974) über die Isomerisierung von Lutein zu Zeaxanthin durch 20-minütiges Erhitzen einer Lösung von Lutein und Kaliummethanolat in methanolischem Dimethylsulfoxid in einem verschlossenen Rohr unter Stickstoff bei 118°C, was eine Ausbeute von nur 10-15% lieferte. Im anschliessenden Artikel (ibid, S. 139-140) zeigten Andrewes, Borch und Liaaen-Jensen, dass es sich bei dieser Isomerisierung um die Umwandlung von (3R,3'R,6'R)-Lutein in (3R,3'S)-Zeaxanthin handelte. Nachteilig bei der Methode von Andrewes war nicht nur die immer noch nicht zufriedenstellende Ausbeute an Zeaxanthin, sondern auch der infolge einer Nacharbeitung etablierte Befund, dass zahlreiche (Z)-Isomere von sowohl Zeaxanthin als auch Lutein erzeugt werden und die Reaktion deshalb nicht-selektiv ist. Zudem stellt eine Isomerisierung, die in einem verschlossenen Reaktionsgefäss erfolgt, keine leicht zu kontrollierende Reaktion dar. Auch wenn die Umwandlung in einem offenen System - unter Argon - durchgeführt wird, stellt es sich heraus, dass nach wie vor unter Verwendung von Kaliummethylat als Base eine unkontrollierbare (E)- → (Z)-Isomerisierung stattfindet.

In letzter Zeit ist eine weitere einschlägige Publikation erschienen, und zwar die PCT-Patentpublikation WO 96/02594. Diese beschreibt ein Verfahren zur Isomerisierung von Lutein zu Zeaxanthin in einer stark alkalischen Lösung unter geregelten Temperatur- und Druckbedingungen. Bei der Base handelt es sich um ein Alkalimetallhydroxid, Calciumhydroxid, Natriumcarbonat, Ammoniumhydroxid oder eine organische Base, insbesondere Ethylamin, Ethanolamin oder Morpholin. Ein anderes Lösungsmittel als Wasser wird nicht verwendet. Durch dieses Verfahren wurde angeblich eine Anreicherung des Zeaxanthins von 4,5% (im Edukt) auf 15,8 bis 24,0% erzielt; allerdings wird nicht erwähnt, wieviel Zersetzungsprodukte bei der Isomerisierung entstanden sind. Auch diese Ausbeute reicht für kommerzielle Zwecke nicht aus.

Ziel der vorliegenden Erfindung ist es, ausgehend von einem die beiden Xanthophylle Lutein und Zeaxanthin (jeweils gegebenenfalls in veresterter Form) enthaltenden natürlichen Produkt oder sogar von reinem oder nahezu reinem Lutein den Gehalt an Zeaxanthin anzureichern bzw. Zeaxanthin herzustellen, und zwar in möglichst grosser Ausbeute. Es wurde nun überraschenderweise gefunden, dass dieses Ziel durch eine ganze besondere Wahl der Reaktionsbedingungen, insbesondere die Wahl des Lösungsmittels und der Base, erreicht werden kann. Durch diese Wahl kann nämlich die Ausbeute an Zeaxanthin entscheidend verbessert werden.

Gegenstand der vorliegenden Erfindung ist ein Verfahren zur Umwandlung von Lutein oder von dessen in der Natur vorkommenden Estern in Zeaxanthin durch basenkatalysierte Isomerisierung, das dadurch gekennzeichnet ist, dass man ein gegebenenfalls vorbehandeltes natürliches Lutein-haltiges Produkt oder reines Lutein in einem Gemisch von einer wässrigen Lösung eines Alkalihydroxids und entweder Dimethylsulfoxid oder einem auf gesättigten aliphatischen und/oder aromatischen Kohlenwasserstoffen basierenden organischen Lösungsmittel bei Temperaturen oberhalb von etwa 50°C erhitzt, wobei im Falle der Verwendung des auf Kohlenwasserstoffen basierenden organischen Lösungsmittels das Verfahren in Gegenwart eines Phasentransferkatalysators durchgeführt wird.

Als natürliches Lutein-haltiges Produkt verwendet man vorzugsweise einen Lutein enthaltenden Rohstoff pflanzlicher Herkunft, welcher gegebenenfalls chemisch, z.B. durch Verseifung, vorbehandelt worden ist. Ein solcher Rohstoff kann ein Konzentrat oder Extrakt sein und liegt dementsprechend in Form eines gemahlenen Pulvers oder eines flüssigen oder harzigen Materials ("oleoresin") vor. Besonders bevorzugt zu diesem Zweck sind Pulver oder Extrakte der gelben oder orangen Blüten von Sammetblumen (Tagetes erecta usw.), die sowohl (hauptsächlich) Lutein als auch Zeaxanthin enthalten. Gemäss F.W. Quackenbush et al. [J.AOAC, 55 (3), 617-621 (1972)] beträgt das Verhältnis Lutein:Zeaxanthin in diesen Rohstoffen etwa 72-88:16-4. Die Xanthophylle kommen dort hauptsächlich als Ester der Palmitin-, Myristin- und Stearinsäure vor [W. Gau et al., J. Chromatography 262, 277-284 (1983)]. Da sich das umzuwandelnde Lutein vorzugsweise in freier Form befindet, werden derartige Ester zweckmässigerweise im voraus zum freien Lutein verseift, bevor der Rohstoff im erfindungsgemässen Verfahren eingesetzt wird, und solche Verseifungen sowie zum selben Zweck durchgeführte enzymatische Hydrolysen sind aus dem Stand der Technik bekannt (siehe beispielsweise US-Patentschrift 3.523.138 bzw. 3.783.099). Pulver (Mehle) und verseifte und nicht verseifte Extrakte von Tagetes sind seit langem kommerziell erhältlich und können als Ausgangsmaterialien für das erfindungsgemässe Verfahren dienen. Beispiele dieser Rohstoffe sind verseifte Tagetesextrakte der mexikanischen Firmen ALCOSA und IOSA. Das ALCOSA-Produkt "pasta saponificada amarillo" ist eine bräunliche Paste mit einem Gehalt an Total-Xantho-phyllen von beispielsweise etwa 2,7%, bestehend aus etwa 92,5% Lutein und etwa 6,6% Zeaxanthin, während das IOSA-Produkt "HI-GOLD 20" ein grünlich-gelbes Pulver ist, welches beispielsweise etwa 0,96% Total-Xanthophylle (etwa 65,5% Lutein und etwa 27,2% Zeaxanthin) enthält (die Prozente sind Flächenprozente). Diese Zahlen wurden infolge der Analyse von bestimmten Produktionschargen (Batches) etabliert und können von Batch zu Batch variieren, was auch für die nachfolgend angegebenen Analysenergebnisse gilt. Weitere Rohstoffe sind "FLORA GLO" [Kemin Ind., Iowa, USA; etwa 739 g/kg Total-Xanthophylle, von denen etwa 681 g/kg aus (all-E)-Lutein und etwa 58 g/kg aus (all-E)-Zeaxanthin bestehen], "ORO GLO Layer dry" (verestert, also nicht verseift; ebenfalls Kemin Ind.; etwa 19,4 g/kg Total-Xanthophylle: etwa 17,6 g/kg Lutein und etwa 1,8 g/kg Zeaxanthin), "HI-GOLD 20 Lutexan" (IOSA; etwa 16,3 g/kg Total-Xanthophylle: etwa 11,0 g/kg Lutein und etwa 5,3 g/kg Zeaxanthin) sowie "XANTOPINA PLUS" (verestert; Bioquimex S.A., Mexico; etwa 351 g/kg Total-Xanthophylle: etwa 330 g/kg Lutein und etwa 21 g/kg Zeaxanthin). Der kommerziell erhältliche Rohstoff kann vor Einsatz gewünschtenfalls bearbeitet werden, um die Xanthophylle aufzukonzentrieren, beispielsweise durch Extraktion, gefolgt von Chromatographie und eventuell auch nach Kristallisation. Eine typische Aufkonzentrierungsmethode besteht darin, den Rohstoff mit Aceton zu extrahieren, den Extrakt einer Flash-Chromatographie an Kieselgel unter Verwendung eines Hexan/Ethylacetat-Gemisches und dann reinen Ethylacetats als Eluierungsmittel zu unterwerfen, die erhaltenen Fraktionen einzuengen und den daraus resultierenden Festkörper zu kristallisieren, z.B. aus einem Gemisch von Methylenchlorid und Methanol. Unter Verwendung dieser Methode konnte man beispielsweise aus 100 g "pasta saponificada amarillo" (ALCOSA; enthaltend etwa 2,7% Total-Xanthophylle) etwa 2,4 g kristallines Material erhalten, das aus etwa 93% (all-E)-(3R,3'R,6'R)-Lutein und etwa 7% (all-E)-(3R,3'R)-Zeaxanthin bestand. Dieses Produkt ist ein Beispiel eines vorbehandelten natürlichen Lutein-haltigen Produktes.

Als Alternative zu Dimethylsulfoxid als (organischem) Lösungsmittel wird im erfindungsgemässen Verfahren ein auf gesättigten aliphatischen und/oder aromatischen Kohlenwasserstoffen basierendes organisches Lösungsmittel verwendet. Es handelt sich dabei insbesondere um ein flüssiges Alkan bzw. einen aromatischen Kohlenwasserstoff, oder ein Gemisch von zwei oder mehreren dieser Kohlenwasserstoffe, z.B. ein Gemisch von mehreren flüssigen Alkanen, von mehreren aromatischen Kohlenwasserstoffen oder von einem oder mehreren solchen Alkanen mit einem oder mehreren aromatischen Kohlenwasserstoffen. Als flüssiges Alkan kommt insbesondere ein geradkettiges oder verzeigtes Alkan mit mindestens 5 Kohlenstoffatomen, vorzugsweise mit 5 bis 10 Kohlenstoffatomen, wie beispielsweise Pentan, Hexan oder Heptan, in Frage. Als Alkangemische eignen sich insbesondere Petrolether, vorzugsweise hochsiedende Petrolether, und als aromatische Kohlenwasserstoffe insbesondere Benzol und Toluol.

Dimethylsulfoxid ist bekanntlich in Wasser löslich, so dass es mit der ebenfalls verwendeten wässrigen Alkalihydroxidlösung leicht eine wässrigorganische Lösung bildet. Im Gegensatz dazu sind die Alkane und aromatischen Kohlenwasserstoffe (nahezu) wasserunlöslich, so dass bei deren Verwendung ein Phasentransferkatalysator eingesetzt werden muss.

Bei dem Alkalihydroxid handelt es sich zweckmässigerweise um Natrium- oder Kaliumhydroxid, vorzugsweise das letztere, dessen Konzentration in der wässrigen Lösung geeigneterweise mindestens 3-molar (M) beträgt. Es können sogar wässrige Lösungen eingesetzt werden, die Konzentrationen bis zur Sättigung aufweisen. Die Konzentration der wässrigen Alkalihydroxidlösung liegt vorzugsweise im Bereich von etwa 7 M bis etwa 14 M.

Beispiele der erfindungsgemäss verwendeten Phasentransferkatalysatoren sind Tricaprylmethylammoniumchlorid (Aliquat® 336), Tetra(n-butyl)ammoniumhydrogensulfat, verschiedene Alkylbenzyldimethylammoniumchloride (Benzalkoniumchlorid), Benzyltri(n-butyl)-ammoniumbromid und Tri(n-butyl)methylammoniumjodid. Vorzugsweise verwendet man als Phasentransferkatalysator Tricaprylmethylammoniumchlorid.

Was die Mengenverhältnisse betrifft, verwendet man pro Mol (berechnetes) Xanthophyll(e) zweckmässigerweise etwa 10 bis etwa 450 Mol Base (Alkalihydroxid) und - falls benötigt - etwa 0,5 bis etwa 5 Mol Phasentransferkatalysator, vorzugsweise etwa 200 bis etwa 250 Mol Base bzw. etwa 0,5 bis etwa 1,5 Mol Phasentransferkatalysator. Das Volumenverhältnis Dimethylsulfoxid:wässrige Alkalihydroxidlösung beträgt im allgemeinen etwa 4:1 bis etwa 1:2, vorzugsweise etwa 2,5:1 bis etwa 1:1. Im Falle der Verwendung eines auf gesättigten aliphatischen und/oder aromatischen Kohlenwasserstoffen basierenden organischen Lösungsmittels beträgt das diesbezügliche Volumenverhältnis organisches Lösungsmittel:wässrige Alkalihydroxidlösung im allgemeinen etwa 8:1 bis etwa 1:2, vorzugsweise etwa 4:1 bis etwa 1:1. Es ergibt sich, dass man unter Berücksichtigung dieser zweckmässigen Mengenverhältnisse sowie anderer Faktoren, insbesondere der Menge und Zusammensetzung der Xanthophylle im gegebenenfalls vorbehandelten natürlichen Lutein-haltigen Produkt bzw. der Menge Lutein selber (Ausgangsmaterial) im allgemeinen etwa 50 bis 300 ml des Lösungsmittel-Alkalihydroxidlösung-Gemisches pro 10 g Xanthophyll-enthaltendes Ausgangsmaterial, vorzugsweise etwa 60 bis 80 ml pro 10 g, verwendet.

Dem Umstand Rechnung tragend, dass bei zu hohen Temperaturen und/oder zu hoher Reaktionsdauer die Bildung von unerwünschten Nebenprodukten und die Zersetzung des erwünschten Zeaxanthins gefördert wird, wird die erfindungsgemässe Umwandlung bei Temperaturen durchgeführt, die möglichst 120°C nicht überschreiten, d.h. die Umwandlung wird zweckmässigerweise bei Temperaturen im Bereich von etwa 50°C bis etwa 120°C durchgeführt. Vorzugsweise wird bei Temperaturen im Bereich von etwa 80°C bis etwa 100°C gearbeitet.

Die Reaktionsdauer hängt unter anderem von der Reaktionstemperatur, der Menge und Konzentration der wässrigen Alkalihydroxidlösung und der Menge und Natur des verwendeten organischen Lösungsmittels ab. Im allgemeinen beträgt diese Dauer etwa 5 bis etwa 65 Stunden. Vorzugsweise dauert die Umwandlung aber nicht länger als etwa 24 Stunden.

Unter den obigen basischen Reaktionsbedingungen wird nicht nur das im Edukt befindliche Lutein in das gewünschte Zeaxanthin in guter Ausbeute umgewandelt, sondern allfällige Ester von Lutein und/oder Zeaxanthin, z.B. die oben erwähnten Palmitin-, Myristin- und Stearinester, werden grösstenteils in freies Zeaxanthin übergeführt.

Die Aufarbeitung des Gemisches nach Beendung der Umwandlung erfolgt auf einfache Weise, und zwar zweckmässigerweise durch Abkühlen des Gemisches auf Raumtemperatur oder etwa 0°C, eventuelle Zugabe eines Alkohols, vorzugsweise Methanol oder Ethanol, und Abfiltration des Festkörpers, welcher hauptsächlich aus angereichertem Zeaxanthin besteht. Zudem können konventionelle Reinigungstechniken, wie beispielsweise Extraktion, Säulenchromatographie und Umkristallisation, verwendet werden. Die letzten drei Behandlungen werden insbesondere im Falle der Verwendung eines Phasentransferkatalysators benötigt. Eine eventuell durchgeführte Umkristallisation erfolgt besonders gut mit einem Gemisch aus Methylenchlorid und Methanol.

Die vorliegende Erfindung wird durch die nachfolgenden Beispiele veranschaulicht:

### Beispiel 1

### Vorbereitung des Ausgangsmaterials

300 g "pasta saponificada amarillo" [Industrias ALCOSA S.A. DE C.V.; 23,9 g/kg (all-E)-Lutein und 3,1 g/kg (all-E)-Zeaxanthin] werden in 1,5 1 Aceton suspendiert, und die Suspension wird 30 Minuten bei Raumtemperatur gerührt. Dann gibt man 200 g Dicalite® (Filterhilfsmittel; Dicalite Europe Nord S.A.) zu und rührt weitere 5 Minuten. Die resultierende Aufschlämmung wird dann durch eine Schicht Dicalite® filtriert, die Dicalite® -Schicht in 780 ml Aceton suspendiert und die neue Suspension 30 Minuten bei Raumtemperatur gerührt. Nach Filtration der Suspension durch eine Dicalite® -Schicht wird diese sechsmal mit je 50 ml Aceton gewaschen. Man engt die vereinigten Filtrate unter vermindertem Druck bei 35°C ein und erhält auf diese Weise 143 g einer dunklen, roten Paste.

Die Paste wird nun an 1 kg Silika (Mesh 70-230) unter Verwendung von 4 Hexan/Ethylacetat (2:1), gefolgt von 1,3 Ethylacetat einer Flash-Chromatographie unterworfen. Man engt die Ethylacetatfraktion unter vermindertem Druck bei 35°C ein, was 20,6 g rotes, halbkristallines Oel ergibt. Dieses Oel wird dann aus einem Methylenchlorid/Methanol-Gemisch kristallisiert und das Kristallisat 6 Stunden unter Hochvakuum bei 37°C getrocknet. Auf diese Weise erhält man 6,36 g eines Gemisches von (all-E)-Lutein und (all-E)-Zeaxanthin (93:6 Flächenprozent) als dunkelrote Kristalle.

### Beispiel 2

### Umwandlung von Lutein in Zeaxanthin

### Allgemeine Vorschrift:

Zu einem Xanthophyll enthaltenden Material [beispielsweise ein gemäss Beispiel 1 erhaltenes Gemisch von Lutein und Zeaxanthin, ein kommerziell erhältliches verseiftes, an Zeaxanthin angereichertes Tagetesextrakt "oleoresina amarillo saponificada" (ALCOSA) oder "HI GOLD 20" (IOSA), oder ein ebenfalls kommerziell erhältliches nichtverseiftes Tagetesextrakt der US-Firma Kemin Industries, Iowa] in einem mit Rückflusskondensator, Rührer und Thermometer ausgestatteten Rundkolben wird das Lösungsmittel Dimethylsulfoxid bzw. flüssiges Alkan oder Alkangemisch gegeben. Im Falle der Verwendung des flüssigen Alkans oder Alkangemisches wird dann der Phasentransferkatalysator zugegeben. Anschliessend gibt man die wässrige Lösung des Alkalihydroxids zu und erwärmt das Reaktionsgemisch. Zur Verfolgung des Reaktionsverlaufs kann man periodisch eine Probe des Reaktionsgemisches (Lösung oder Suspension) entnehmen, diese mit Methylenchlorid verdünnen, die Methylenchloridlösung mit gesättigter wässriger Ammoniumchloridlösung und dann mit Wasser bis zur Neutralität waschen, die Methylenchloridlösung über wasserfreiem Natriumsulfat trocknen und schliesslich die Lösung einer HPLC-Analyse unterziehen.

Nach festgestellter Beendigung der Reaktion (keine weitere Umwandlung Lutein → Zeaxanthin) wird die homogene oder heterogene Lösung unter Rühren auf Raumtemperatur oder 0°C abgekühlt, wobei man gewünschtenfalls einen Alkohol, z.B. Methanol oder n-Propanol, während des Rührens und Abkühlens zugibt. Man filtriert die resultierende Suspension durch ein Papier- oder Glasfaserfilter, wäscht den Festkörper mehrmals mit dem ausgewählten Alkohol, trocknet ihn unter Hochvakuum bei etwa 37°C mindestens eine Stunde und kristallisiert ihn gewünschtenfalls aus Methylenchlorid/Methanol oder Methylenchlorid/Hexan um.

### Prozedur und Ergebnisse mit dem Ausgangsmaterial "oleoresina amarillo saponificada":

Das Ausgangsmaterial enthält 23,9 g/kg Lutein und 3,1 g/kg Zeaxanthin. Nach Extrahieren, Chromatographie und Kristallisation erhält man ein Gemisch, das zu 93,1% aus Lutein, zu 6,6% aus Zeaxanthin sowie zu 0,5% aus weiterem Material besteht. Die Ergebnisse der gemäss der obigen Vorschrift durchgeführten Umwandlung sind in der nachfolgenden Tabelle 1 zusammengefasst:

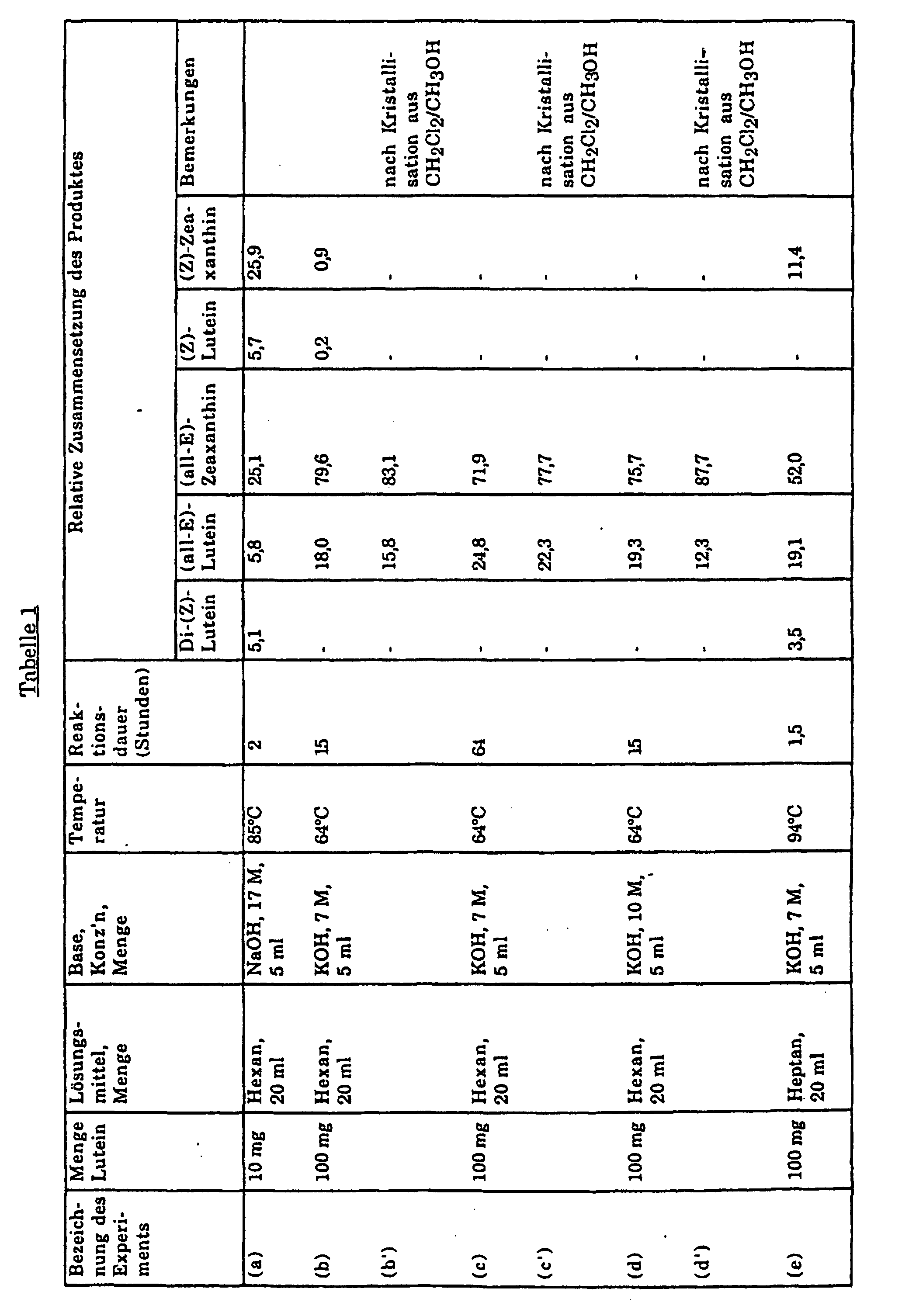

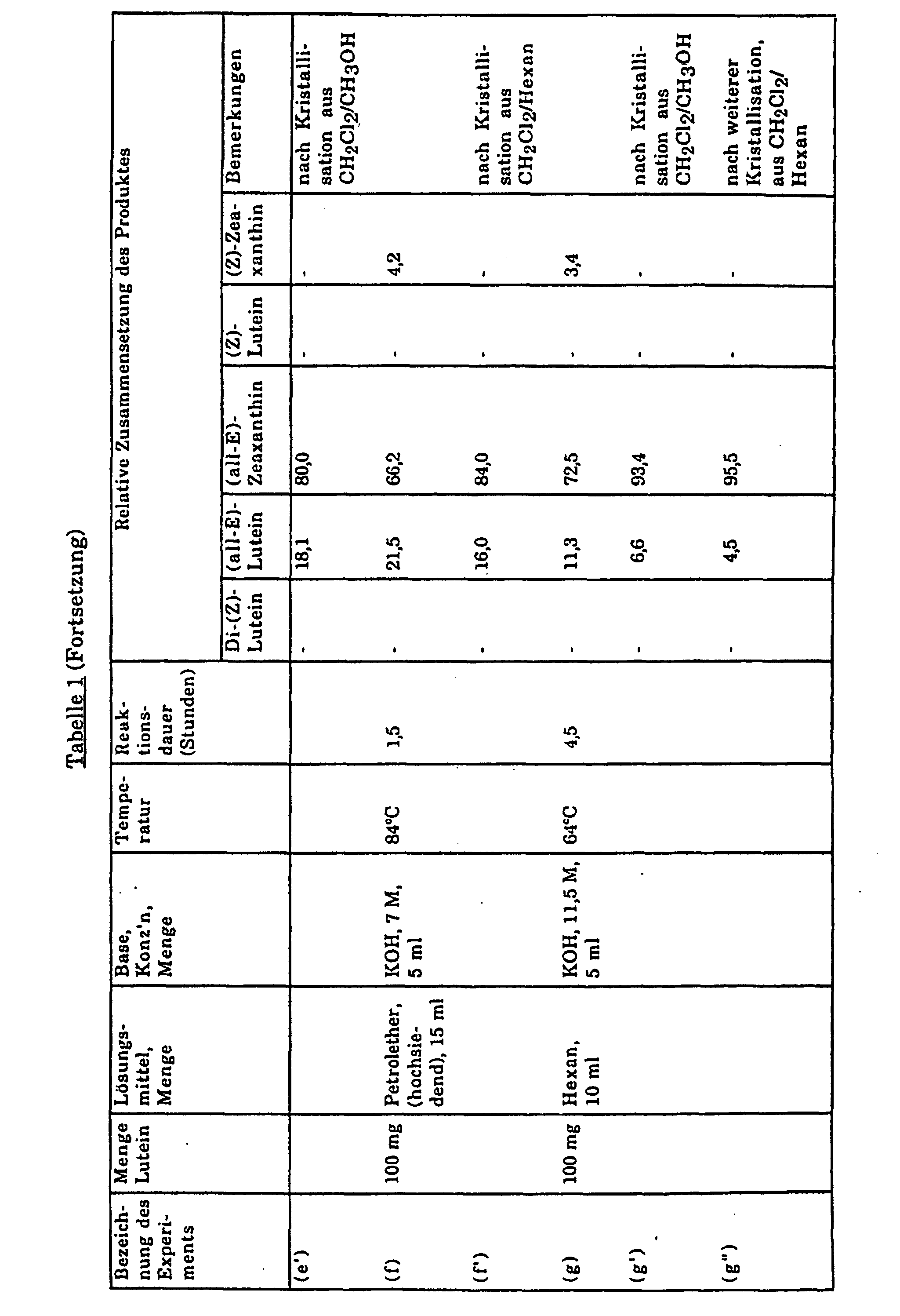

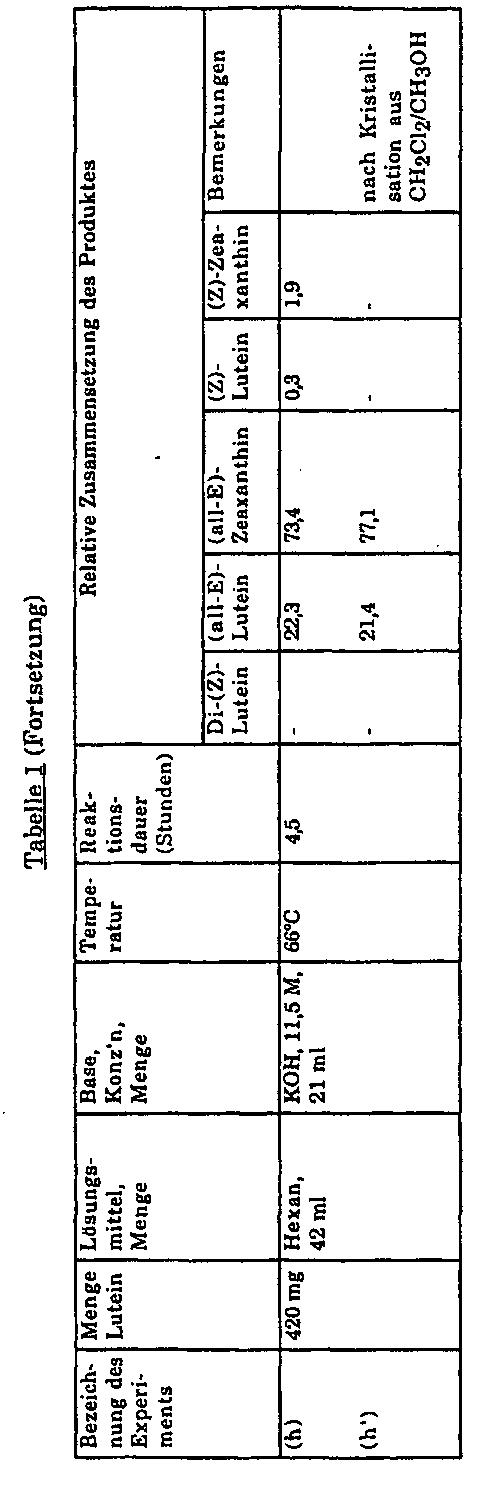

### Weitere Bemerkungen:

- In allen Fällen mit Ausnahme der Experimente (h) und (h') werden dem Reaktionsgemisch 100 mg Aliquat® 336 (Fluka Chemie AG, Buchs, Schweiz) als Phasentransferkatalysator zugesetzt, da als organisches Lösungsmittel Hexan, Heptan oder Petrolether verwendet wird; in den Experimenten (h) und (h') werden 300 mg Aliquat® 336 zugesetzt.
- Im Falle des Experiments (a) handelt es sich bei der Temperatur um diejenige des Heizbads; in allen anderen Fällen wird die tatsächliche Temperatur des Reaktionsgemisches angegeben.

### Prozedur und Ergebnisse mit dem Ausgangsmaterial "oleoresina amarillo saponificada" (direkte Verwendung)

Das Ausgangsmaterial weist die obige Zusammensetzung auf und wird nicht extrahiert, chromatographiert und kristallisiert, sondern unmittelbar eingesetzt. Die Ergebnisse der gemäss der obigen Vorschrift durchgeführten Umwandlung (ohne Phasentransferkatalysator) sind in der nachfolgenden Tabelle 2 zusammengefasst:

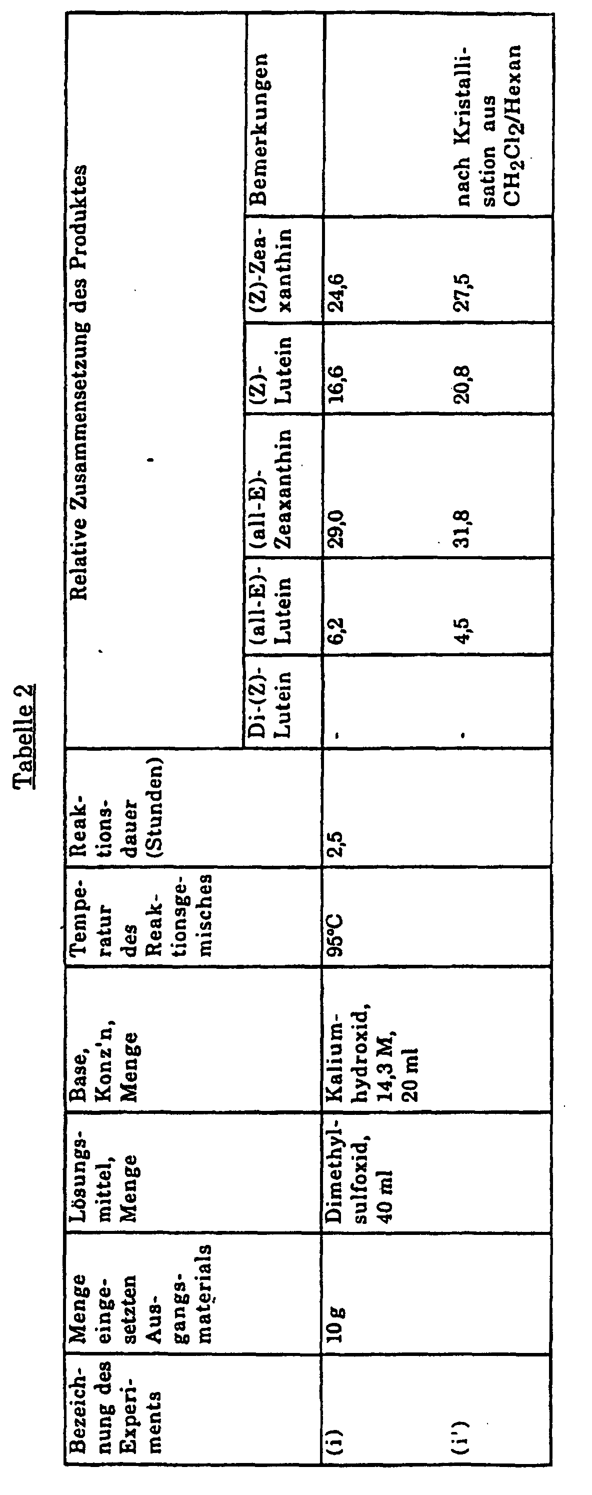

### Prozedur und Ergebnisse mit dem Ausgangsmaterial FLORA GLO

Das Ausgangsmaterial weist eine relative (prozentuale) Zusammensetzung von 91,3% (all-E)-Lutein und 6,6% (all-E)-Zeaxanthin und einen Totalgehalt an Xanthophyllen von 73,9% (739 g/kg Rohware) auf. Dieses Material ist schon verseift, so dass die Wahrscheinlichkeit von Nebenreaktionen gering ist. Gemäss der obigen Vorschrift (ohne Phasentransferkatalysator) wird die Rohware in einem Gemisch von Dimethylsulfoxid (DMSO) und konzentrierter wässriger Kalilauge (KOH) bei etwa 80°C oder oberhalb dieser Temperatur gerührt. Es fällt auf, dass die Reaktion sehr sauber, d.h. praktisch von Edukt zu Produkt, abläuft. Auch ist die Aufarbeitung der Reaktionslösung einfach: aus der auf 0°C abgekühlten Lösung kristallisiert das Lutein-Zeaxanthin-Gemisch aus und wird leicht abfiltriert und mit Wasser nachgewaschen, um Spuren überschüssiger Base grösstenteils zu entfernen. Die Ergebnisse der auf diese Weise durchgeführten Umwandlung sind in der nachfolgenden Tabelle 3 zusammengefasst:

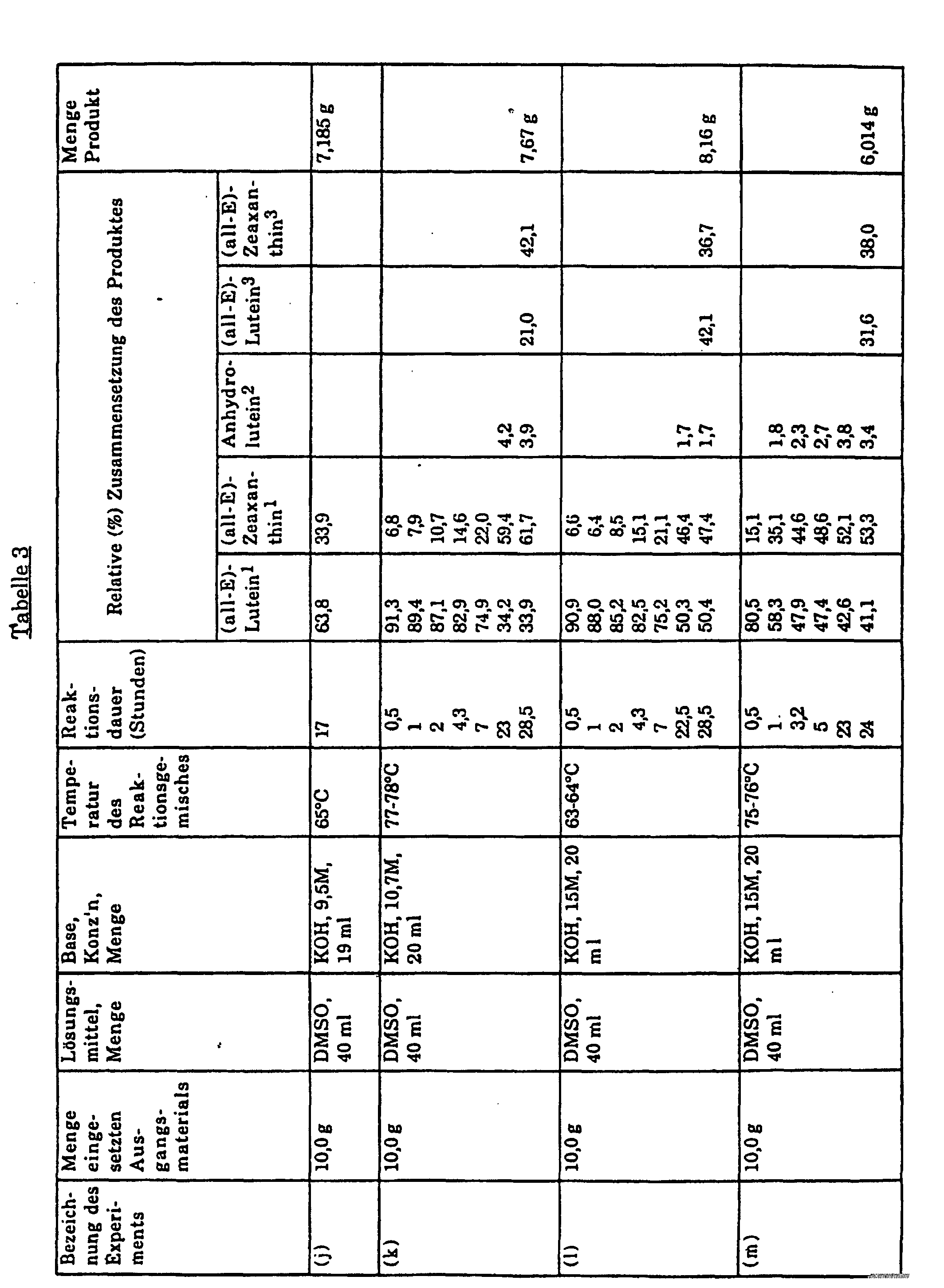

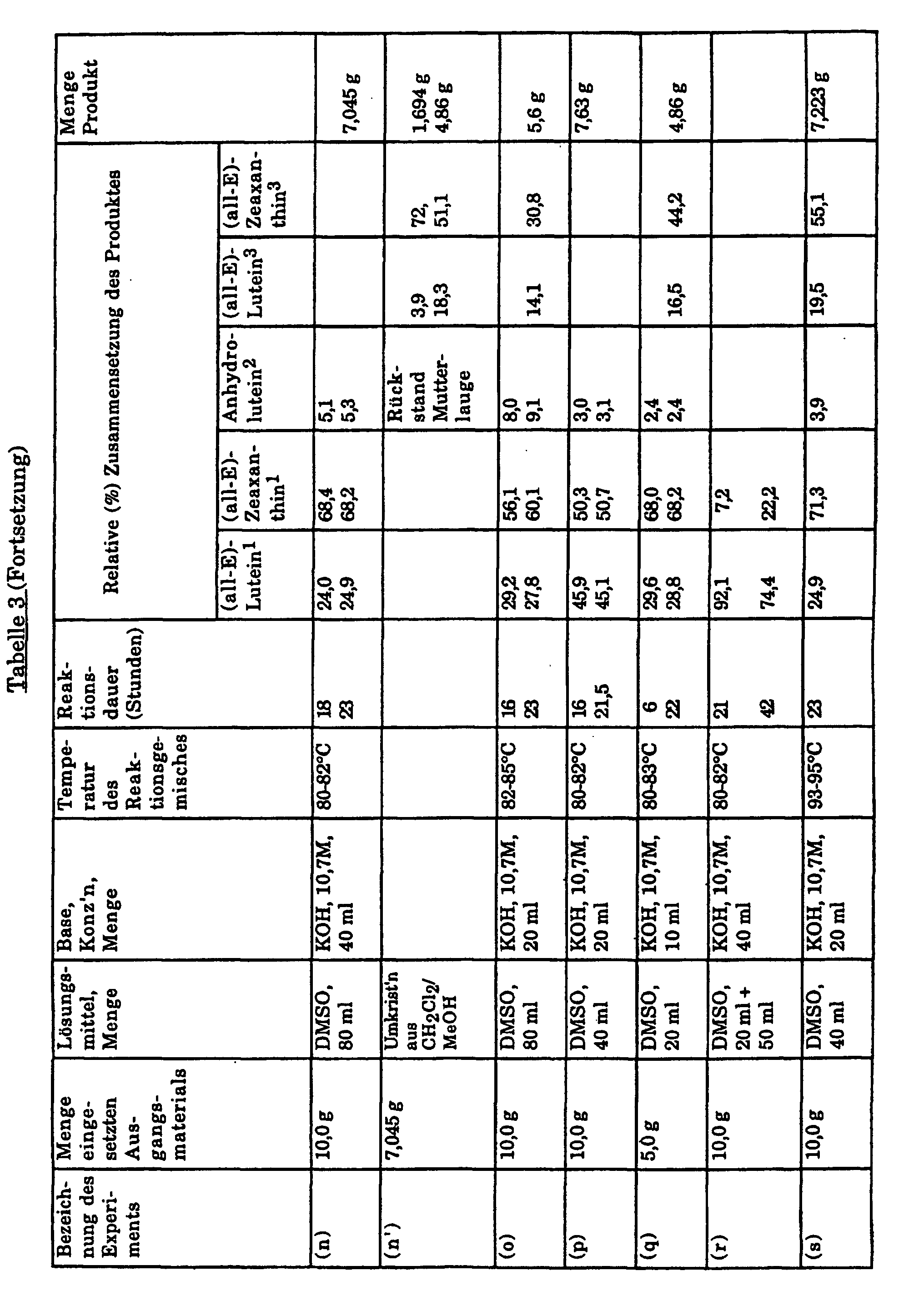

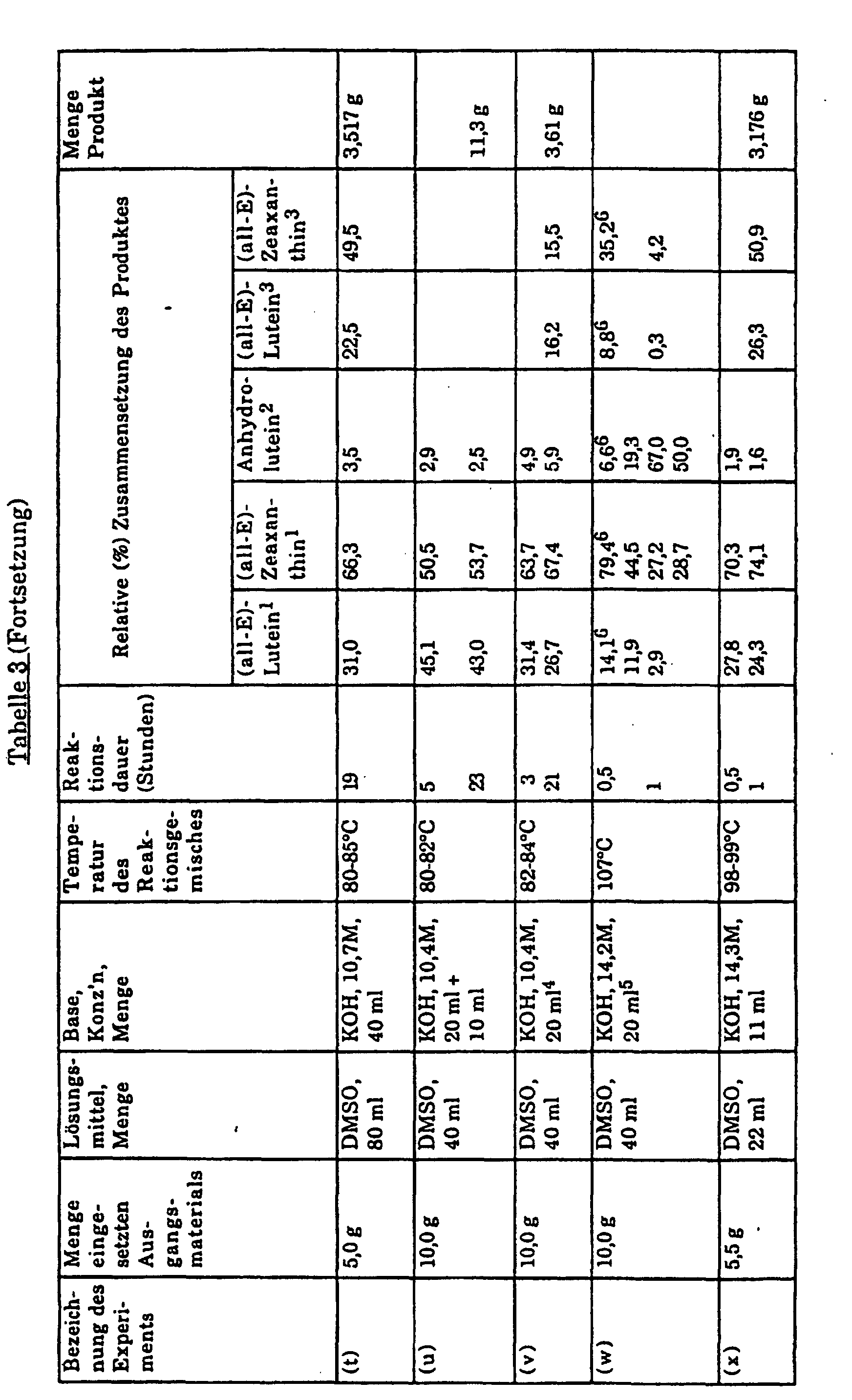

¹ HPLC-Flächenprozent bei 450 nm
² Es wurde nicht bestimmt, um welche Anhydroluteine und andere Carotinoide es sich bei dieser Fraktion handelt
³ Resultate der quantitativen HPLC-Analyse
⁴ Zugabe der Base erst, nachdem die DMSO-FLORA GLO-Suspension 80°C erreicht hat.
⁵ Zugabe der Base erst, nachdem die DMSO-FLORA GLO-Suspension 90°C erreicht hat.
⁶ Der obere Wert wurde jeweils nach 0,5 und 1 Stunde durch Zugabe eines Aliquots der Reaktionslösung zu Methanol, anschliessendes Rühren der Suspension, Abfiltration und Analyse mittels HPLC erhalten. Bestimmung des unteren Wertes wie bereits beschrieben.

### Prozedur und Ergebnisse mit dem Ausgangsmaterial XANTOPINA PLUS

Das Ausgangsmaterial weist eine Zusammensetzung von 90,9% Lutein und 5,3% Zeaxanthin in veresterter Form auf; der Rest besteht aus (Z)-Isomeren von Luteinestern und wenig Anhydroluteinestern. Der Xanthophyllgehalt von XANTOPINA PLUS macht 35,1% aus. Die Ergebnisse der gemäss der obigen Vorschrift durchgeführten Umwandlung (ohne Phasentransferkatalysator) sind in der nachfolgenden Tabelle 4 zusammengefasst:

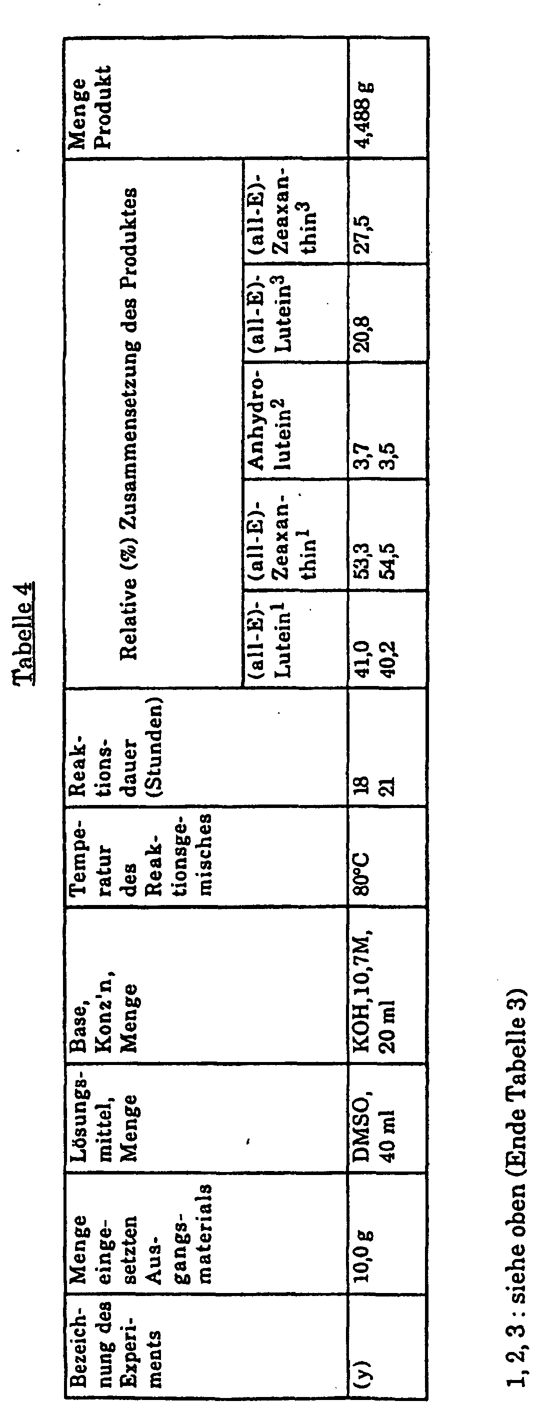

## Patentansprüche

1. Verfahren zur Umwandlung von Lutein oder von dessen in der Natur vorkommenden Estern in Zeaxanthin durch basenkatalysierte Isomerisierung, **dadurch gekennzeichnet, dass** man ein gegebenenfalls vorbehandeltes natürliches Lutein-haltiges Produkt oder reines Lutein in einem Gemisch von einer wässrigen Lösung eines Alkalihydroxids und entweder Dimethylsulfoxid oder einem auf gesättigten aliphatischen und/oder aromatischen Kohlenwasserstoffen basierenden organischen Lösungsmittel bei Temperaturen oberhalb von etwa 50°C erhitzt, wobei im Falle der Verwendung des auf Kohlenwasserstoffen basierenden organischen Lösungsmittels das Verfahren in Gegenwart eines Phasentransferkatalysators durchgeführt wird.

2. Verfahren nach Anspruch 1, **dadurch gekennzeichnet, dass** als natürliches Lutein-haltiges Produkt ein Lutein enthaltender Rohstoff pflanzlicher Herkunft, welcher gegebenenfalls chemisch, z.B. durch Verseifung, vorbehandelt worden ist, verwendet wird.

3. Verfahren nach Anspruch 2, **dadurch gekennzeichnet, dass** als natürliches Lutein-haltiges Produkt ein Pulver oder Extrakt der gelben oder orangen Blüten von Sammetblumen verwendet wird.

4. Verfahren nach einem der Ansprüche 1 bis 3, **dadurch gekennzeichnet, dass** als auf Kohlenwasserstoffen basierendes organisches Lösungsmittel Pentan, Hexan, Heptan, Petrolether, Benzol oder Toluol, oder ein Gemisch von zwei oder mehreren dieser Lösungsmittel, verwendet wird.

5. Verfahren nach einem der Ansprüche 1 bis 4, **dadurch gekennzeichnet, dass** als Alkalihydroxid Natrium- oder Kaliumhydroxid, vorzugsweise Kaliumhydroxid, verwendet wird.

6. Verfahren nach einem der Ansprüche 1 bis 5, **dadurch gekennzeichnet, dass** die Konzentration der wässrigen Alkalihydroxidlösung mindestens 3M beträgt.

7. Verfahren nach Anspruch 6, **dadurch gekennzeichnet, dass** die Konzentration der wässrigen Alkalihydroxidlösung etwa 7M bis etwa 14M beträgt.

8. Verfahren nach einem der Ansprüche 1 bis 7, **dadurch gekennzeichnet, dass** der Phasentransferkatalysator Tricaprylmethylammoniumchlorid ist.

9. Verfahren nach einem der Ansprüche 1 bis 8, **dadurch gekennzeichnet, dass** die Umwandlung bei Temperaturen im Bereich von etwa 50°C bis etwa 120°C durchgeführt wird.

10. Verfahren nach Anspruch 9, **dadurch gekennzeichnet, dass** die Umwandlung bei Temperaturen im Bereich von etwa 80°C bis etwa 100°C durchgeführt wird.

## Claims

1. A process for the conversion of lutein or of its esters which occur in nature into zeaxanthin by base-catalyzed isomerization, which process comprises heating an optionally pre-treated natural lutein-containing product or pure lutein in a mixture of an aqueous solution of an alkali hydroxide and either dimethyl sulphoxide or an organic solvent based on saturated aliphatic and/or aromatic hydrocarbons at temperatures above about 50°C, with the process being carried out in the presence of a phase transfer catalyst when an organic solvent based on hydrocarbons is used.

2. A process according to claim 1, wherein a lutein-containing raw material of plant origin, which has optionally been pre-treated chemically, e.g. by saponification, is used as the natural lutein-containing product.

3. A process according to claim 2, wherein a powder or extract of the yellow or orange blossoms of marigolds is used as the natural lutein-containing product.

4. A process according to any one of claims 1 to 3, wherein pentane, hexane, heptane, petroleum ether, benzene or toluene, or a mixture of two or more of these solvents, is used as the organic solvent based on hydrocarbons.

5. A process according to any one of claims 1 to 4, wherein sodium hydroxide or potassium hydroxide, preferably potassium hydroxide, is used as the alkali hydroxide.

6. A process according to any one of claims 1 to 5, wherein the concentration of the aqueous alkali hydroxide solution is at least 3M.

7. A process according to claim 6, wherein the concentration of the aqueous alkali hydroxide solution is about 7M to about 14M.

8. A process according to any one of claims 1 to 7, wherein the phase transfer catalyst is tricaprylmethylammonium chloride.

9. A process according to any one of claims 1 to 8, wherein the conversion is carried out at temperatures in the range of about 50°C to about 120°C.

10. A process according to claim 9, wherein the conversion is carried out at temperatures in the range of about 80°C to about 100°C.

## Revendications

1. Procédé pour la conversion en zéaxanthine de lutéine ou de ses esters existant dans la nature, par isomérisation catalysée par des bases, **caractérisé en ce qu'**on chauffe à des températures supérieures à environ 50°C de la lutéine pure ou un produit naturel contenant de la lutéine, éventuellement prétraité, dans un mélange d'une solution aqueuse d'un hydroxyde de métal alcalin et soit de diméthylsulfoxyde, soit d'un solvant organique à base d'hydrocarbures aliphatiques saturés et/ou d'hydrocarbures aromatiques, dans le cas de l'utilisation du solvant organique à base d'hydrocarbures, le procédé étant effectué en présence d'un catalyseur de transfert de phase.

2. Procédé selon la revendication 1, **caractérisé en ce qu'**on utilise comme produit naturel contenant de la lutéine une matière première d'origine végétale contenant de la lutéine, qui a été éventuellement prétraité chimiquement, par exemple, par saponification.

3. Procédé selon la revendication 2, **caractérisé en ce qu'**on utilise comme produit naturel contenant de la lutéine une poudre ou un extrait des fleurs jaunes ou orangées de tagetes.

4. Procédé selon l'une quelconque des revendications 1 à 3, **caractérisé en ce qu'**on utilise comme solvant organique à base d'hydrocarbures le pentane, l'hexane, l'heptane, l'éther de pétrole, le benzène ou le toluène, ou un mélange de deux ou plus de deux de ces solvants.

5. Procédé selon l'une quelconque des revendications 1 à 4, **caractérisé en ce qu'**on utilise comme hydroxyde de métal alcalin l'hydroxyde de sodium ou de potassium, de préférence l'hydroxyde de potassium.

6. Procédé selon l'une quelconque des revendications 1 à 5, **caractérisé en ce que** la concentration de la solution aqueuse d'hydroxyde de métal alcalin est au moins 3 M.

7. Procédé selon la revendication 6, **caractérisé en ce que** la concentration de la solution aqueuse d'hydroxyde de métal alcalin va d'environ 7 M à environ 14 M.

8. Procédé selon l'une quelconque des revendications 1 à 7, **caractérisé en ce que** le catalyseur de transfert de phase est le chlorure de tricapryl-méthylammonium.

9. Procédé selon l'une quelconque des revendications 1 à 8, **caractérisé en ce que** la conversion est effectuée à des températures dans la plage d'environ 50°C à environ 120°C.

10. Procédé selon la revendication 9, **caractérisé en ce que** la conversion est effectuée à des températures dans la plage d'environ 80°C à environ 100°C.
